# EUROPEAN PATENT SPECIFICATION

(11) **EP 4 606 324 B1**
(45) Date of publication and mention of the grant of the patent: **02.12.2026**
(21) Application number: 25188528.1
(22) Date of filing: 04.12.2017
(51) Int. Cl.: A61B 17/072, A61B 17/115, A61B 17/11, A61B 17/00

(54) **INSTRUMENT FOR PERFORMING ANASTOMOSIS**
INSTRUMENT ZUR DURCHFÜHRUNG EINER ANASTOMOSE
INSTRUMENT POUR RÉALISER UNE ANASTOMOSE

(30) Priority: 02.12.2016 NL 2017917
(43) Date of publication of application: 27.08.2025
(62) Document number(s) of the earlier application(s) in accordance with Art. 76 EPC: 17817288.8 / 3 547 935
(73) Proprietor: Implican B.V., 9713 GX Groningen (NL); Rijksuniversiteit Groningen, 9712 CP Groningen (NL); Academisch Ziekenhuis Groningen, 9713 GZ Groningen (NL)
(72) Inventor: HAVENGA, Klaas, 9713 GX Groningen (NL); NIEUWENHUIS, Jan, 9713 GX Groningen (NL); BOESJES, Olf, 9713 GX Groningen (NL)
(74) Representative: V.O.

(56) References cited:
- EP-A1- 2 272 442
- EP-A2- 2 258 282
- EP-A2- 2 620 104
- WO-A1-2009/096822
- DE-A1- 102011 055 236
- US-A- 4 700 703
- US-A1- 2010 270 356

## Description

The present disclosure is directed to a surgical instrument and a method for connecting and fixating bodily tissues. The present invention is directed to a surgical instrument for performing anastomosis of the gastro-intestinal tract.

Anastomoses, *i.e.* the joining of two sections of tubular organ sections of the gastro-intestinal tract such as the esophagus, colon or other parts, are frequently associated with complications such a leakage, infections, fibrosis and the like. For instance, anastomotic leakage is observed in about 10% of low anterior resection cases involving anastomosis of the colorectal segment by conventional circular staplers.

Favorable results in terms of preventing complications after anastomosis have been obtained by using compression anastomosis (see *e.g.* Kaider-Person et al., The American Journal of Surgery (2008) 195, 818-826). Conventional compression anastomosis includes for instance clamping and fixating the two tissue layers of the gastro-intestinal tract between two members of a clamping device such as the known Murphy button, Boerema knot, Valtrac^{™} or magnetic rings (see Cossu et al., The American Surgeon (2000) (8),759-762 and Jansen et al. Surgery, Gynecoloy & Obstetrics (1981) 153, 537-545 respectively).

In spite of the favorable results, compression anastomosis is still posed by challenges and therefore the use of conventional surgical staplers is generally standard in anastomosis procedures. Conventional surgical staplers give no compression on the connected bowel segments and preserve the circulation of blood between the staples. It is accordingly desirable to further improve compression anastomosis so it will *i.a.* become easier to perform and more accessible. It is desired to improve compression anastomosis in terms of the healing process such to reduce post-operational complication.

US 2010/270356 A1 discloses a surgical instrument.

The tissue of the gastro-intestinal tract is a tubular layered structure basically comprising three layers of different tissue types. The peripheral layer primarily comprises muscularis, the subsequent submucosal layer primarily comprises collagen and the luminal layer primarily comprises mucosa. The present inventors realized that optimal conditions for wound healing of the anastomosis involves contacting and fixating the submucosal layers of the gastro-intestinal tract sections that are to be connected thereby allowing the tissue to heal by primary intent. Healing by primary intent may increase the healing process rate and reduce the risk of leakage, infections, fibrosis and the like.

In order to contact the submucosal layers of the gastro-intestinal tract sections, the submucosal layers of the gastro-intestinal tract sections that are to be connected are preferably stripped from the adjacent muscularis and/or mucosal layers. The present inventors surprisingly realized that this may be achieved by placing a part of the two gastro-intestinal tract sections that are to be connected on top of each other and by then compressing the two gastro-intestinal tract sections between a pressure area to crush the muscularis and/or mucosal layers, followed by increasing said pressure area such that the muscularisis and/or mucosal layers are pressed aside and the submucosal layers are concomitantly stripped from the muscularis and/or mucosal layers and both submucosal layers contact each other.

Accordingly, the present disclosure provides a method and a surgical instrument for performing anastomosis of a first gastro-intestinal tract section to a second gastro-intestinal tract section, both gastro-intestinal tract sections comprising two superficial layers (e.g. a muscularis layer and a mucosal layer) and an intermediate layer (e.g. a submucosal layer). Said method comprises contacting the respective superficial (muscularis) layers of said gastro-intestinal tract sections and compressing the contacted sections between a first pressure area and a second pressure area, wherein the sum of the first pressure area and the second pressure increases during the compression such that the superficial layers are pressed aside and the intermediate layers are contacted.

The present disclosure further provides a surgical instrument, in particular a surgical stapler, said instrument comprising:
- a first body having a first pressure area;
- a second body having a second pressure area;
- a connection means connecting the first body and the second body, said connection means configured to move the first body between an open position away from the second body and a closed position near the second body; wherein the first and second bodies are configured to compress the first gastro-intestinal tract section and the second gastro-intestinal tract section between the first pressure area and the second pressure area, wherein the sum of the first pressure area and the second pressure increases during the compression.

The present inventors surprisingly found that anastomosis methods can be improved by stripping the submucosal layers from the muscularis and/or mucosal layers while the first gastro-intestinal tract section and the second gastro-intestinal tract section are compressed. The method of the present disclosure enables this compressing and stripping by increasing the sum of the total pressure area during the compression. The compression is thus initiated with a smaller total pressure area compared to the final total pressure area.

The surgical instrument of the present invention is defined in claim 1. Further embodiments of the invention are recited in the dependent claims.

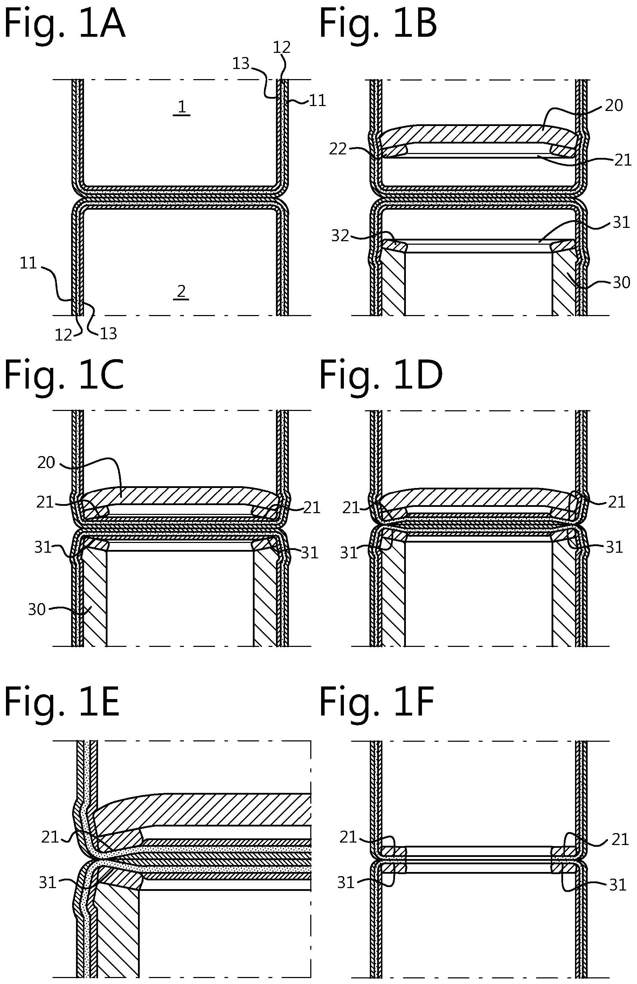

Figure 1A-F show a schematic illustration of a particular embodiment of the method to operate the surgical instrument of the present disclosure.

Figure 1A illustrates the first gastro-intestinal tract section and the second gastro-intestinal tract section, each comprising a muscularis layer (11) and a mucosal layer (13) *(i.e.* the superficial layers) in additional to a submucosal layer (12) *(i.e.* the intermediate layer). The muscularis layers (11) of each gastro-intestinal tract section are contacted. In figure 1B, the first body (20) and second body (30) of the surgical instrument are illustrated being present in the first and second gastro-intestinal tract section, respectively. The first gastro-intestinal tract section and the second gastro-intestinal tract section are compressed in between the first body 20 comprising the first pressure area 21 and the second body 30 comprising the second pressure area 31 (figure 1C) during which the sum of the first pressure area and the second pressure area increases during the compression (figure 1F illustrates a higher sum of the first pressure area and the second pressure area than figure 1E). During compression, the muscularis layers (11) and muscosal layers (13) are pressed aside (figure 1D and figure 1E) and the submucosal layer 12 are contacted (figure 1F). The muscularis layers (11) and muscosal layers (13) may be pressed aside towards the lumen of the gastro-intestinal tract sections (as shown in figure 1D and figure 1E) or towards the bowel *(i.e.* outside the gastro-intestinal tract sections, not shown).

A portion of the first and the second gastro-intestinal tract sections can be removed, e.g. by cutting with a knife, such that the internal lumen of the first and second gastro-intestinal tract sections are connected and the anastomosis is complete (figure 1F).

In a particular embodiment of the present invention, as for instant illustrated in figures 1A-F, the first pressure area is inclined or slanted with respect to the second pressure area. This results in that the sum of the first pressure area and the second pressure area increases during the compression. In other words, during use, the first pressure area and/or second pressure area are slanted or inclined with respect to the first and second gastro-intestinal tract sections that are compressed.

By contacting the submucosal layers, a watertight seal is provided that *i.a.* limits bacterial contamination at the site of contact and a healing process by primary intent is promoted.

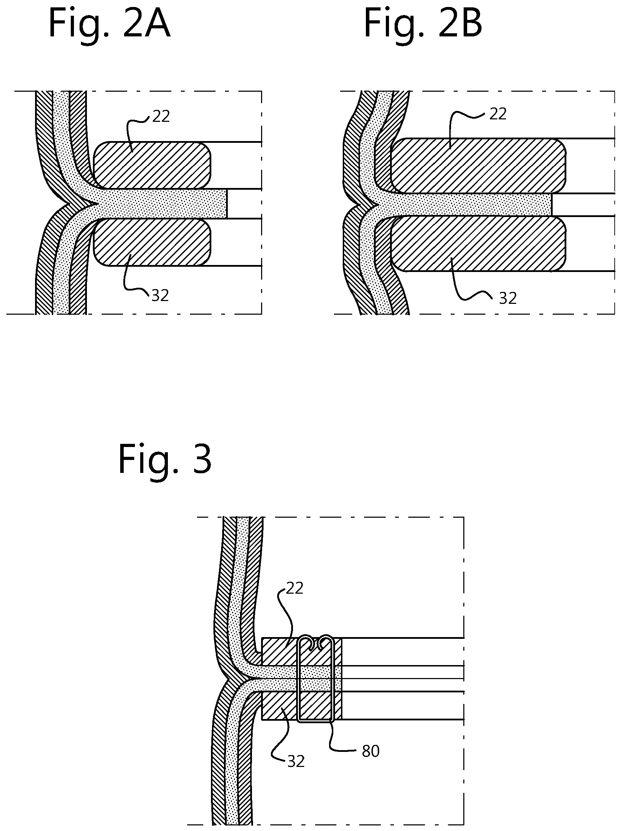

In a preferred embodiment, as illustrated in figures 2A-B, the method to operate the surgical instrument further comprises applying a swellable hydrogel (22, 32) on the part of the compressed first gastro-intestinal tract section and the second gastro-intestinal tract section (figure 2A) to be connected and sealed, fixating said swellable hydrogel onto at least part of the contacted gastro-intestinal tract section and allowing the hydrogel to swell so to exert a force on the sealed gastro-intestinal tract sections (figure 2B). The hydrogel may swell due to the absorption of water or other fluids and this swelling, of for instance about 150 vol%, may results in additional submucosa being contacted *(cf.* figures 2A and 2B) without compromising vascularization of these additionally contacted submucosal layers 11. Sufficient vascularization of the bowel layers promotes the healing process.

The additional contact of the respective submucosal layers of the first and second gastro-intestinal tract sections as for instance shown in figures 2A and 2B, can typically be achieved by providing the hydrogel in two rings which each have an outer diameter that approximate the inner diameter of the first and second gastro-intestinal tract sections in which the two rings are to be placed. The swelling of the hydrogel results in the exertion of an outwardly directed force which result in additional intersubmucosal contact.

The swellable hydrogel may comprise known hydrogel such as swellable polyurethanes and the like. The swellable hydrogel is typically capable of absorbing moisture to swell in an amount of 0-100 vol%, preferably 10-80 vol%, more preferably about 50 vol% with respect to the original volume of the hydrogel. Swelling of the hydrogel after it has been placed on the layers to be sealed improve the sealing and healing process.

Swellable hydrogels are known for medical applications. Caló and Khutoryanskiy, European Polymer Journal 65 (2015) 252-267 discloses hydrogel of which some are capable of swelling, for instance the hydrogels disclosed in EP0524718. Additional suitable hydrogels for this purpose may comprise *e.g*. polyethylene glycol and polybutylene terephthalate multiblock copolymer such as commercially available under the tradename PolyActive ^{™} and disclosed by Bos et al., Pharmaceutical Technology October 2001 p. 110-120, or thermoplastic polyurethanes based on aliphatic hydrophilic polyether such as commercially available under the tradename Tecophilic^{™} and disclosed by Verstraete et al., International Journal of Pharmaceutics 2016, 15, 214-221.

In figure 3, a particular embodiment of the hydogel 22,32 is schematically illustrated wherein the hydrogel 22,32 is fixated by staples 80.

Suitable means for fixation of the hydrogel and thus the first and second gastro-intestinal tract sections comprise staples sutures, glue and/or rivets..

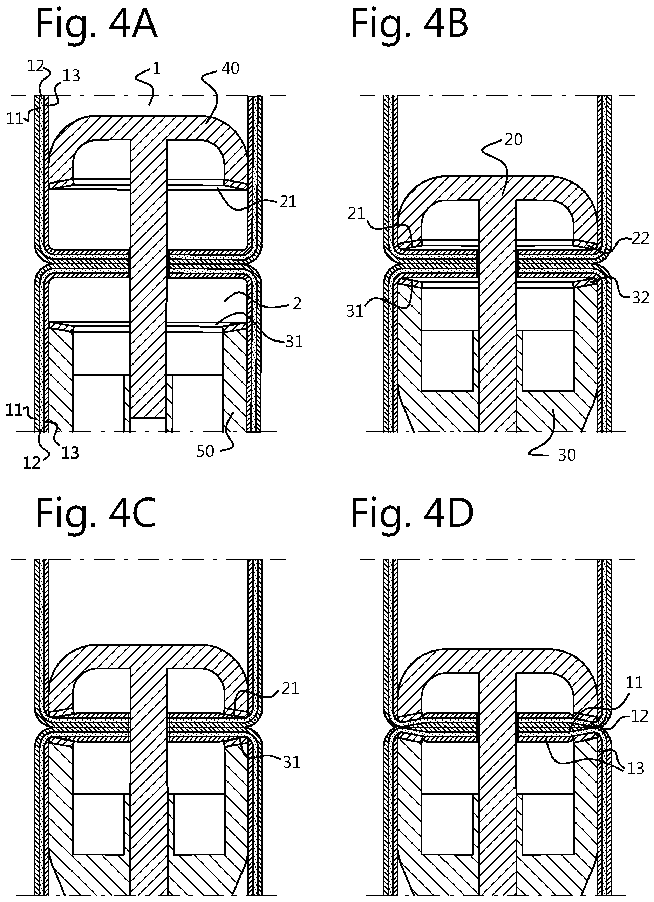

In figures 4A-F, a particularly preferred embodiment of the present invention is illustrated, wherein the first gastro-intestinal tract section and the second gastro-intestinal tract section are compressed by using a surgical stapler comprising an anvil (40) comprising the first pressure area 21 and a casing 50 comprising the second pressure area 32. The surgical stapler is generally applied in an open position (figure 4A) in the lumen of the gastro-intestinal tract, wherein the anvil 20 is placed in the first gastro-intestinal tract section (*e.g*. the proximal part of the bowel) and the casing 30 is placed in the second gastro-intestinal tract section (*e.g*. the distal part of the bowel).

The hydrogel 22,32 may also be provided on the surgical stapler.

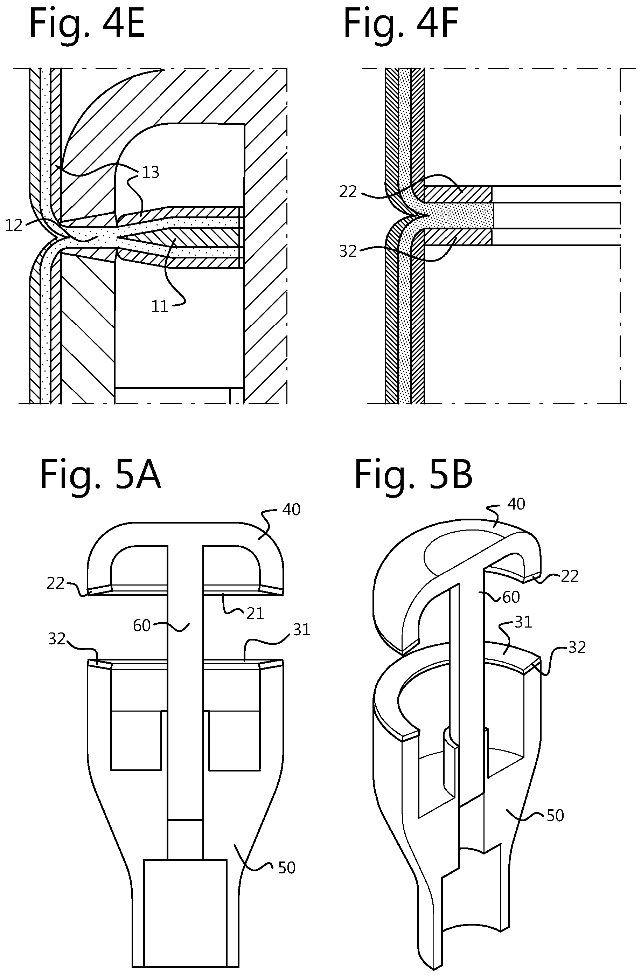

During use of the surgical stapler, the first body is moved from the open position way from the second body, towards a closed position near the second body (figure 4B) such that the compression of the first gastro-intestinal tract section and the second gastro-intestinal tract section may commence (figure 4C). The sum of the first pressure area and the second pressure area increases during the compression *(cf.* figures 4B-E). In figure 4B, the sum of the first pressure area and the second pressure area comprises the initial points of contact of the slanted first pressure area and the second pressure area. In figure 4F, the sum of the first pressure area and the second pressure area is larger than that sum in figure 4B.

During compression the superficial layers (*e.g*. muscularis layers (11) and muscosa layers (13)) are pressed aside and the intermediate layers are contacted (figure 4D) up to a point wherein a substantially watertight sealing between the intermediate layers (*e.g*. submucosal layers 12) is obtained (figure 4E).

After the compression by the surgical stapler is completed, a section of the first gastro-intestinal tract section and the second gastro-intestinal tract section can be removed, *e.g*. by cutting with a knife that is provided in the stapler (not shown), such that the lumen of the first gastro-intestinal tract section and the second gastro-intestinal tract section are connected (figure 4F). This cutting with a knife is also common for conventional circular staplers.

Preferably, the sum of the first pressure area and the second pressure area increases in the direction of the nearest cutting section of the tubular section, *i.e.* in the direction of the nearest part of the knife during cutting, *e.g*. in the direction of the center of the cross-section of the stapler in case the stapler is a circular stapler (*i.e.* towards the lumen of the bowel). This prevents the accumulation of debris, bacteria and other undesired matter ending up near the wound that will have to heal at the periphery of the pressure areas (site of contact of the submucosal layers of the connected gastro-intestinal tract sections) since this debris, bacteria and the like are pushed towards the lumen of the bowel which continuity is restored. Since, the lumen of the bowel generally comprises many bacteria and debris, it is less harmless to push the debris and bacteria towards and into the lumen than in the direction of the outside of the bowel *(i.e.* into the abdominal cavity).

A particular advantage of the present invention is that the swellable hydrogel may be placed and fixated on the contacting intermediate layers (*e.g*. by staples or other means for fixation) and that swelling of the swellable hydrogel may further press aside the mucosa and musculari, without compromising vascularization, thereby facilitating the water tightness of the submucosa connection and healing by primary intent.

The means for fixation and the swellable hydrogel are typically released from the patient's body by necrosis of the submucosa.

In a preferred embodiment, the first body and/or second body of the surgical instrument comprise a swellable hydrogel. The swellable hydrogel may be positioned on the first and/or second body such that during uses, the first gastro-intestinal tract section and the second gastro-intestinal tract section can at least partially be compressed between the swellable hydrogel.

In the case that the swellable hydrogel is present, at least part of the swellable hydrogel surface area is part of the first and/or second pressure area in between which the gastro-intestinal tract sections are compressed. In a particular embodiment, the total pressure area may entirely be comprised by the swellable hydrogel. In another embodiment, the swellable hydrogel may provide only part of the total pressure area in between which the gastro-intestinal tract sections are compressed.

The swellable hydrogel may for instance be fixated by fixation means to the first and/or second body. Examples of such means include for instance pins that protrude from the first and/or second body into the swellable hydrogel. Additionally or alternative, the hydrogel and/or the first and/or second body may also comprises adhesive properties such that the hydrogel loosely adheres to the first and/or second body. The fixation of the swellable hydrogel to the first and/or second body may be releasable or non-releasable.

A non-releasable fixation, which may be preferred, may for instance be achieved by fixating the hydrogel at a section which is removed together with part of the section of the first gastro-intestinal tract section and the second gastro-intestinal tract section which can be removed, *e.g*. by cutting with a knife (*vide supra*). In other words, the hydrogel is also cut by the knife and the part that is fixated to the the first and/or second body can be removed together with the the first and/or second body and the sections of the first and second gastro-intestinal tract section that are to be removed.

For instance, in a particular embodiment, wherein the surgical stapler is a circular surgical stapler and the hydrogel is comprised in one or two rings (*vide infra*), and wherein the circular surgical stapler comprises a circular knife, the inner diameter of the hydrogel-comprising ring may be smaller than the diameter of the circular knife such that after the hydrogel has been fixated by the means for fixation of the hydrogel (*e.g*. staples and/or rivets). The circular surgical stapler may further be configured such that the knife can create a cut to separate the part of the hydrogel-comprising ring that is fixated to the circular surgical stapler and the part of the hydrogel-comprising ring that has been fixated to the gastro-intestinal tract sections. The cut may also be created before the hydrogel-comprising ring that has been fixated to the gastro-intestinal tract sections by maintaining the position ring in between the anvil and the casing of the surgical stapler.

Conventional surgical instruments such as surgical staplers are known under the tradenames Endopath^{™} and DST Series^{™} EEA^{™} Staplers, by Ethicon and Medtronic respectively, and for instance disclosed in US2005059996 and US6503257.

The conventional staplers are not capable of contacting the submucosal layers of the bowel. The conventional staplers are typical configured such that during operation and stapling, as small distance (of about 1.5 to 2 mm) remains in between the anvil and stapler casing. This results in a conventionally stapled anastomosis that heals via secundam (secondary intent).

The surgical instrument of the present invention enables contacting of the submucosal layers and providing a watertight sealing between the contacted submucosal layers. This enable healing of the anastomosis wound via a primary healing process. The first and second bodies of the present surgical instrument are shaped or configured in such a manner that the total pressure area in between which the gastro-intestinal tract sections on which anastomosis is performed are compressed *(i.e.* sum of the first pressure area and the second pressure area), increases during the compression. By providing such a shape that the compression commences with a relatively small total pressure area, the superficial layers *(i.e.* muscularis layers and muscosa layers) that cover the intermediate layers are compressed and pushed away. During further compression, these superficial layers are pushed even further away due to the increasing total pressure area. The total area of the exposed submucosal layers thus increases and is allowed to be contacted and fixated as such.

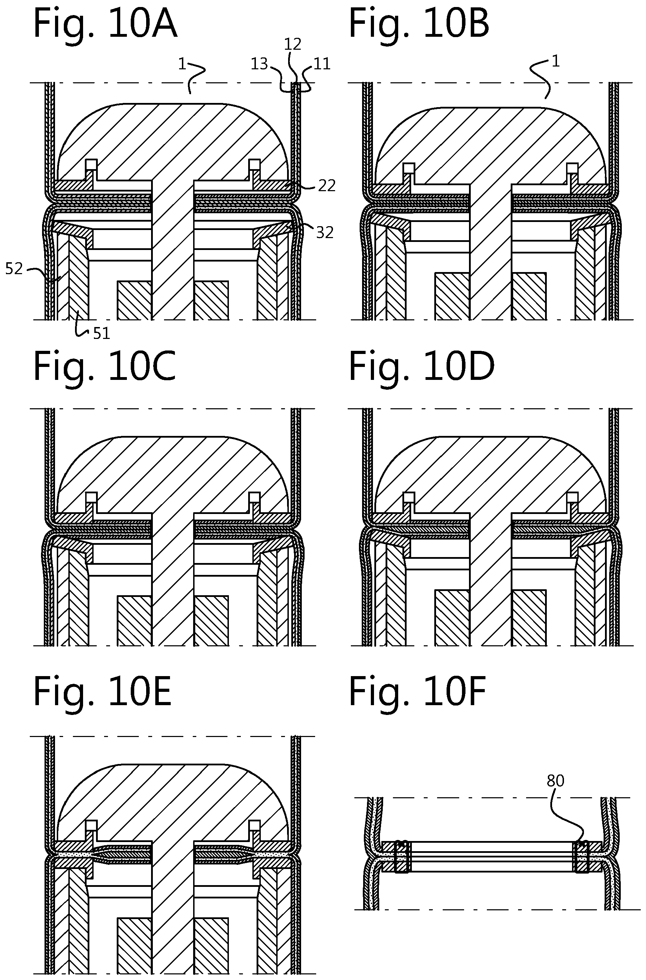

In a particular embodiment, the first and/or second body are configured such that during use, the first gastro-intestinal tract section and the second gastro-intestinal tract section are initially compressed between an inclined first pressure area and/or inclined second pressure area (*e.g.* as shown in figures 4A-F and 10A-F).

In a particularly preferred embodiment the surgical instrument comprises a surgical stapler wherein the first body comprises an anvil and the second body comprises a casing that comprises a staple cartridge and a stapler base through which staples can be driven against the anvil.

Figures 5A and 5B illustrates a cross-section (5A) and a cutthrough (5B) a particular embodiment of the surgical stapler in accordance with the present invention. The anvil 40 comprising the first pressure area 21 is connected by the connecting means 60 (*e.g.* a telescopic shaft comprising screw thread) to the casing 50 that comprises the second pressure area 31. The parts of the anvil and the casing comprising respectively the first and second pressure areas may comprise swellable hydrogel 22, 32.

The present surgical stapler can be operated as described herein-above and illustrated in figure 4A-F. The hydrogel is typically resistibly compressible to a certain extent, such that the intermediate (submucosa) layers can be contacted and fixated over the entire sum of the first and second pressure area *(cf.* figures 4D and 4F) such that an optimal sealing is obtained.

The inventors surprisingly found that leakage of the anastomosis may partially be the result of the microbiome. Without wishing to be bound by theory, certain bacteria (*e.g*. enterococcus faecalis) are believed to be capable of excreting enzymes capable of dissolving collagen. (see e.g.Van Praagh et al. Surgical Endoscopy (2016) 30, 2259-2265; Shogan et al. Science Translational Medicine (2015) 7, 286ra68) To limit or prevent the proliferation of such bacteria, it may be preferred that the hydrogel comprises one or more antibiotics. Suitable antibiotics may include polymyxin E, tobramycin, amphotericin B, vancomycin and gentamicine. (see *e.g.* Schardey et al. Annals of Surgery (1997) 225, 172-180; Roos et al. British Journal of Surgery (2013) 100, 1579-1588)

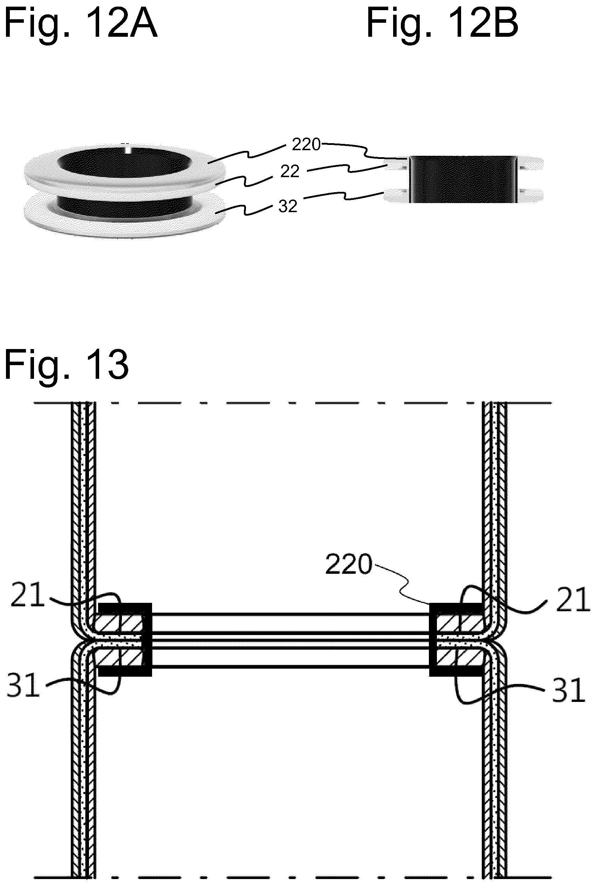

In a preferred embodiment, that is illustrated figure 12A-B, the surgical instrument comprises a cover on at least part of the surface of the swellable hydrogel that does not contact the gastro-intestinal sections after application sealing. As such that delivery of the antibiotics is directed to the sealed tissue sections of the tissue of the gastro-intestinal tract and a higher dosage of antibiotics can be delivered. Preferably, entire said surface of the hydrogel is covered by the cover, such that the hydrogel is essentially entirely surrounded by the cover and the gastro-intestinal sections, as illustrated in figure 13.

Similar to conventional surgical stapler, the casing 50 may comprise a staple cartridge and a stapler base through which staples can be driven against the anvil. Alternatively or additionally, the casing may comprise rivets cartridge and one or more drivers to drive the rivets through the sealed intermediate layers (submucosa) and the optionally present hydrogel.

In a particular embodiment, the anvil of the comprises surgical stapler an inner anvil part having an inner anvil pressure area and an outer anvil part having an outer anvil pressure area, said inner anvil pressure area and outer anvil pressure area being part of the first pressure area, wherein the inner anvil part is configured to be independently movable from the outer anvil part such that the first gastro-intestinal tract section and the second gastro-intestinal tract section can be compressed first between the outer anvil pressure area and at least part of the second pressure area and subsequently further compressed between the inner anvil pressure area and at least part of the second pressure area.

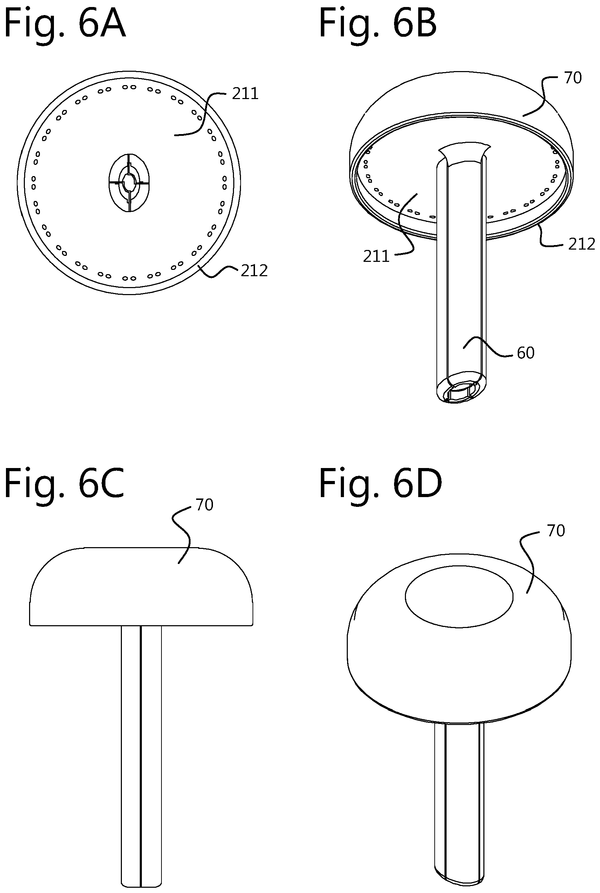

Figure 6A-D illustrates part of a particular embodiment of the anvil comprising the inner anvil part comprising the inner anvil pressure area 211 and further the outer anvil part 70 comprising the outer anvil pressure area 212. The rim of the outer anvil part that comprises the outer anvil pressure area 212 is typically about 1 mm in width. The width may vary depending on the desired application of the surgical stapler comprising the anvil.

The inner and outer anvil parts may be connected by connecting means 60, which also connect the anvil to the casing (not shown). In this particular embodiment, the connecting means 60 may be configured such that the inner anvil can slide independently of the outer anvil such that the inner anvil pressure area 211 and the outer anvil pressure area 212 can be joined to form a single pressure area. During operation, the first gastro-intestinal tract section and the second gastro-intestinal tract section are first compressed in between the outer anvil pressure area 212 and the second pressure area (not shown) to press the superficial tissue layers aside and connect the intermediate (submucosa) layers. Subsequently, by sliding the inner anvil part such that the inner anvil pressure area 211 will join the outer anvil pressure area 212, the sum of the first pressure area and the second pressure area will increase, sliding more of the superficial tissue layers aside and enable full contact between the intermediate layers.

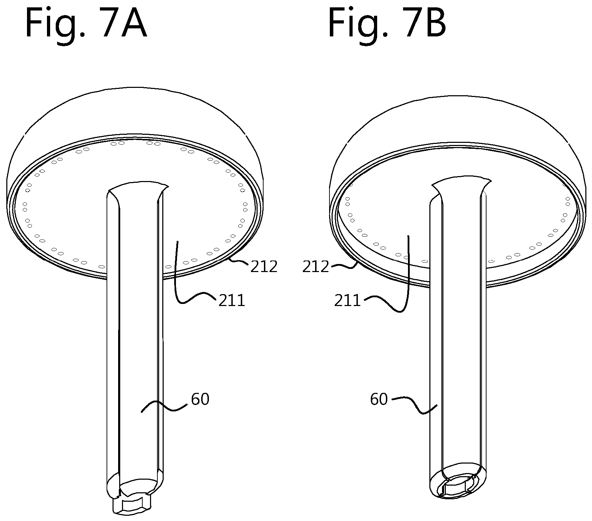

Figures 7A and 7B is a further illustration of the anvil comprising the inner and outer anvil parts. Figure 7B shows the connecting means 60 in a sliding position such that the inner anvil pressure area 211 and the outer anvil pressure area 212 are not joined to form a single pressure area. In Figure 7A, the connecting means 60 in a sliding position such that the inner anvil pressure area 211 and the outer anvil pressure area 212 are joined to form a single pressure area.

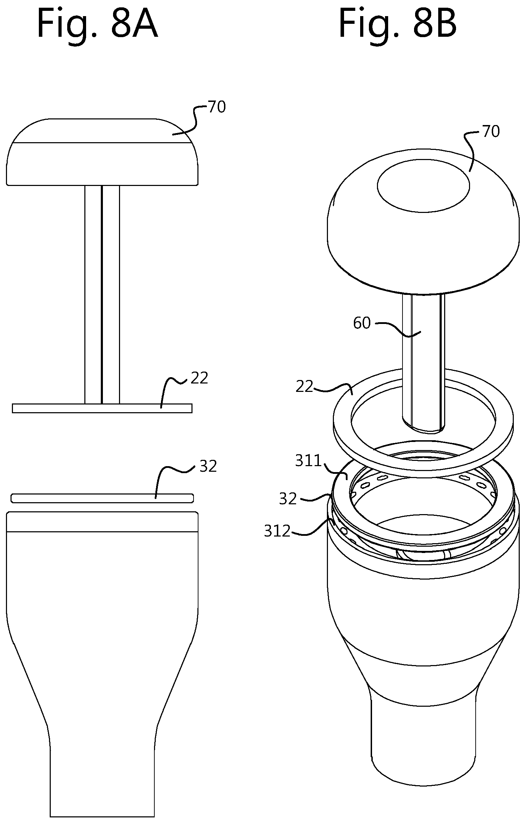

In the embodiments wherein the anvil comprises an inner and outer anvil part, the anvil may also further comprise the swellable hydrogel. Figures 8A and 8B illustrates part of a particular embodiment of the surgical stapler comprising two rings of the swellable hydrogel. Figure 8A illustrates the cross-section and figure 8B illustrates a perspective view. One ring of the swellable hydrogel 21 falls within the outer anvil part onto the inner anvil part. The other ring of the swellable hydrogel 22 falls within part of the casing that comprises a protruding rim. The ring of the swellable hydrogel 22 comprising pressure area 311 may form the second pressure area together with pressure area 312.

In another particular embodiment the stapler base of the surgical stapler comprises an inner stapler base part having an inner stapler base pressure area and an outer stapler base part having an outer stapler base pressure area, said inner stapler base pressure area and outer stapler base pressure area being part of the second pressure area, wherein the inner stapler base part is configured to be independently movable from the outer stapler base part such that the first gastro-intestinal tract section and the second gastro-intestinal tract section is first compressed between the outer stapler base pressure area and at least part of the first pressure area and subsequently further compressed between the inner stapler base pressure area and at least part of the first pressure area.

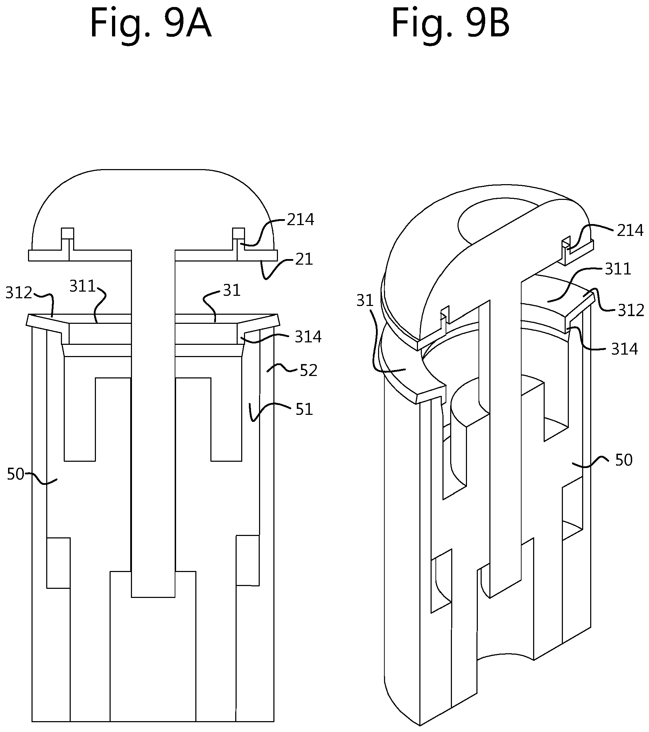

Figures 9A and 9B illustrates a cross-section (9A) and a cutthrough (9B) of a particular embodiment of casing 50 that comprise the stapler base comprising the inner stapler base part 51 and the outer stapler base part 52. For illustration purposes, a particular anvil is also show, but the illustrated stapler base may also be combined with another anvil (*e.g.* with the anvil comprising the inner anvil part and the outer anvil part as described herein above). In addition, the optionally present hydrogel 31 is illustrated. In case the hydrogel 31 is present, the inner stapler base pressure area 311 and the outer stapler base pressure area 312 may be part of the hydrogel 30. The stapler base may be configured such that the inner stapler base part 51 can slide independently of the outer anvil such that the inner stapler base pressure area 311 and the outer stapler base pressure area 312 can be joined to form a single pressure area.

Figures 10A-F illustrate the operation of the particular embodiment of the invention described above wherein the stapler base comprises the inner stapler base part 51 and the outer stapler base part 52. Similar to described herein-above, the stapling instrument may be operated such that the first gastro-intestinal tract section and the second gastro-intestinal tract section having layers 11, 12 and 13 are first compressed in between the first pressure area 21 and the outer stapler base pressure area 312 to press the superficial tissue layers aside and connect the intermediate (submucosa) layers *(cf.* figures 10A to 10D). Subsequently, by sliding the inner stapler base part such that the inner stapler base pressure area 311 will join the outer stapler pressure area 312, the sum of the first pressure area and the second pressure area will increase, sliding more of the superficial tissue layers aside and enable full contact between the intermediate layers *(cf.* figure 10E). After fixation the hydrogel rings 22 and 32 with for instance staples 80, a knife (not shown) may cut away a doughnut shaped section of the first gastro-intestinal tract section and the second gastro-intestinal tract section such that the fixed tract section remain ((*cf.* figure 10F).

For the embodiments wherein the anvil and/or staple base comprise inner and outers parts, it is preferred that it protrudes from the outer edges of the anvil and/or casing. For instance, in case the surgical instrument is a surgical circular stapler, the outer diameter of the hydrogel rings 21, 31 is preferably larger than the outer diameter of the anvil and/or casing, whichever comprises inner and outers parts. The protrusion of the hydrogel from the edge of the anvil and/or staple base reduces the risk that the hydrogel is pushed inwards by the outer parts upon compression. This greatly increases overall reliability of the instrument and the method of the present disclosure.

The surgical instrument according to the present invention typically further comprises a knife for creating a cut in a cutting section of the gastro-intestinal tract sections. It is preferred that the first body and/or the second body are further configured such that during use, the sum of the first pressure area and the second pressure area increases in the direction of the nearest cutting section of the tubular gastro-intestinal tract sections, *e.g.* in the direction of the center of the cross-section of the circular stapler which is the lumen of the bowel. After cutting, part of gastro-intestinal tract section may be removed to connect the lumen of the first and second gastro-intestinal tract sections. By configuring the first and the second body such that the sum of the pressure area increased towards the cutting section, the debris, bacteria and other undesired material will be pushed towards the lumen of the bowel and will thus not interfere at the site of primary wound healing as described herein above. This facilitates healing of the anastomosis wound.

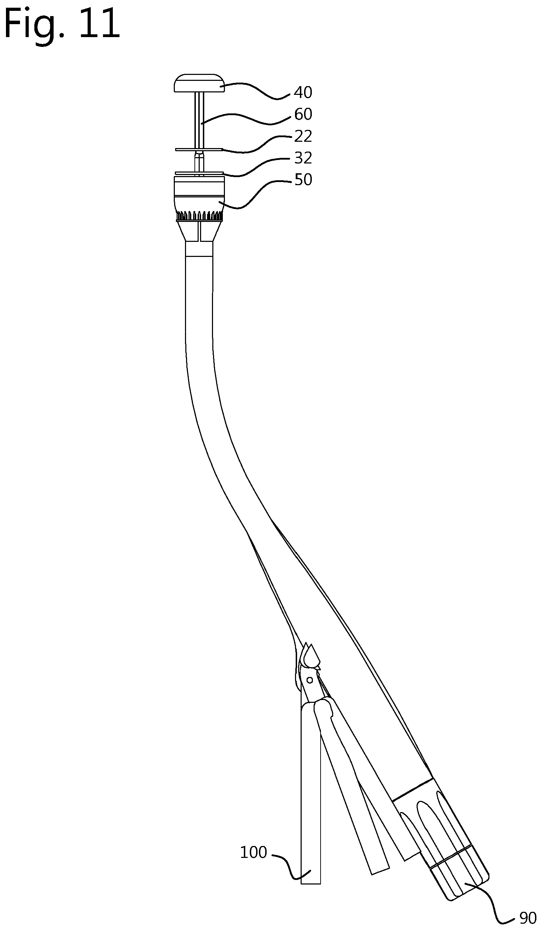

Figure 11 illustrates a particular embodiment of the surgical circular stapler of the present invention. The surgical circular stapler comprises anvil 40 and casing 50 that are connected by the connecting means 60. The surgical circular stapler may further comprise rings of swellable hydrogel 22,32.

In a preferred embodiment of the surgical circular stapler, one or both of the swellable hydrogel rings comprises a raised edge 224,324 at the inner diameter of the ring *(cf.* figures 9A , 9B). This raised edge may fixate, *e.g.* by using an adhesive and/or clamping, the swellable hydrogel ring to the anvil or the casing. After the hydrogel ring is placed on the gastro-intestinal tract section and fixated by *e.g.* staples, the knife may create a cut in between the raised edge and the outer diameter of the hydrogel ring such that inner part of the hydrogel ring comprising the raised edge can be removed together with the doughnut part of gastro-intestinal tract section that is removed.

The connection means 60 connecting the anvil 40 and the casing 60 is configured to move the anvil between an open position away from the casing and a closed position near the casing. The moving between the open and closing position can for instance be carried out by turning the adjusting means 90. The compression may be carried out by continuation of turning the adjusting means 90 after the anvil is brought to the close position. After the anvil is brought in the desired position relative to the casing, lever 100 may be operated to fixate the contacted gastro-intestinal tract sections

The surgical stapler of the present invention may be configured such that in the closed position that anvil is at a minimal, predetermined, distance, e.g. about 1.4 mm, from the casing. Typically, submucosal layers of bowel section are slightly thicker than 1.4 mm. Configuring the surgical stapler such that the distance between the anvil and the casing can not be less than a minimal distance (*e.g*. 1.4 mm) will prevent undesired overcompression of the submucosal layers and cutting or tearing of these layers. However, the thickness of submucosal layers may be different for each patient.

It is preferred that the surgical stapler of the present invention may be adapted such that the force of compression is indicated. The advantage of such a configuration is that excessive compression pressures that may cause the pressure area to crush and destroy the submucosal layers can be avoided.

In a preferred embodiment, the compression of the surgical instrument may be obtained in a staged and revisable manner. Preferable, a partially compressed stapler may be released such that the operating surgeon may reposition the stapler, *e.g*. to release any tension on one of the gastro-intestinal tracts if necessary.

Figures 4-11 illustrate particular embodiments of a surgical circular stapler. However, the surgical stapler of the present invention is not limited to surgical circular staplers and may also comprise surgical linear staplers.

## Claims

1. Surgical instrument for performing anastomosis of a first gastro-intestinal tract section and a second gastro-intestinal tract section, said instrument comprising:
- a first body (20) having a first pressure area (21);
- a second body (30) having a second pressure area (31);
- connection means (60) connecting the first body and the second body,
wherein the connection means is configured to move the first body between an open position away from the second body and a closed position near the second body;
wherein the first and second bodies are configured such that, during use, the first gastro-intestinal tract section and the second gastro-intestinal tract section are compressed between the first pressure area and the second pressure area such that the sum of the first pressure area and the second pressure area increases during the compression to seal the first gastro-intestinal tract section and the second gastro-intestinal tract section
**characterized in that** the first body and second body are configured such that the sum of the first pressure area and the second pressure area increases during the compression such that the muscularis layer and/or mucosal layer of the gastro-intestinal tract sections are pressed aside and the submucosal layers of the gastro-intestinal tract sections are contacted.

2. Surgical instrument according to claim 1, wherein the first body and/or second body comprises a swellable hydrogel (22, 32).

3. Surgical instrument according to claim 2, configured such that the swellable hydrogel is configured to placed where the first gastro-intestinal tract section and the second gastro-intestinal tract section are contacted and sealed, such that the hydrogel on swelling exerts a force on the sealed sections and results in additional contact between the submucosal layers without compromising vascularization of these additionally contacted submucosal layers to promote healing by primary intent.

4. Surgical instrument according to any of claims 2-3, wherein the swellable hydrogel is capable swelling in an amount of 0-100 vol%, preferably 10-80 vol%, more preferably about 50 vol% with respect to the original volume of the hydrogel.

5. Surgical instrument according to any of the preceding claims, wherein the first and/or second body are configured such to compress the first gastro-intestinal tract section and the second gastro-intestinal tract section between an inclined first pressure area and/or inclined second pressure area.

6. Surgical instrument according to any of the preceding claims, said surgical instrument being a surgical stapler wherein the first body comprises an anvil (40) and the second body comprises a casing (50) that comprises a staple cartridge and a stapler base through which staples can be driven against the anvil.

7. Surgical instrument according to any of the preceding claims, further comprising a knife for creating a cut in a cutting section of the tubular gastro-intestinal tract sections;
wherein the first body and/or the second body are further configured such that during use, the sum of the first pressure area and the second pressure area increases in the direction of the cutting section of the tubular gastro-intestinal tract sections.

8. Surgical instrument according to any of the preceding claims , wherein said surgical stapler is a surgical circular stapler or a surgical linear stapler.

9. Surgical instrument according to any of the preceding claims, wherein the surgical instrument is configured for stripping the submucosal layers from the muscularis and/or mucosal layers while the first gastro-intestinal tract section and the second gastro-intestinal tract section are compressed.

10. Surgical instrument according to any of the preceding claims, wherein the surgical instrument is configured to press the muscularis layers (11) and muscosal layers (13) of the gastro-intestinal tract sections aside towards the lumen of the gastro-intestinal tract sections.

11. Surgical instrument according to any of claims 1-9, wherein the surgical instrument is configured to press the muscularis layers (11) and muscosal layers (13) of the gastro-intestinal tract sections aside towards the bowel of the gastro-intestinal tract sections.

12. Surgical instrument according to any of the preceding claims, wherein during use the first pressure area and/or second pressure area can be positioned slanted or inclined with respect to the first and second gastro-intestinal tract sections that are to be compressed.

13. Surgical instrument according to any of the preceding claims, during use, during compression, the muscularis layers (11) and muscosa layers (13) are pressed aside and the submucosal layers are contacted up to a point wherein a substantially watertight sealing between the submucosal layers is obtained.

14. Surgical instrument according to any of the preceding claims, wherein the surgical instrument is a circular stapler, and during use the sum of the first pressure area and the second pressure area increases in the direction of the center of the cross-section of the stapler.

## Patentansprüche

1. Chirurgisches Instrument zum Durchführen einer Anastomose eines ersten Gastrointestinaltraktabschnitts und eines zweiten Gastrointestinaltraktabschnitts, wobei das Instrument umfasst:
- einen ersten Körper (20) mit einer ersten Druckbereich (21);
- einen zweiten Körper (30) mit einer zweiten Druckbereich (31);
- Verbindungsmittel (60), die den ersten Körper und den zweiten Körper verbindet, wobei die Verbindungsmittel dazu ausgebildet sind, den ersten Körper zwischen einer offenen Position weg von dem zweiten Körper und einer geschlossenen Position nahe dem zweiten Körper zu bewegen;
wobei der erste und der zweite Körper derart ausgebildet sind, dass während der Verwendung der erste Gastrointestinaltraktabschnitt und der zweite Gastrointestinaltraktabschnitt zwischen der ersten Druckbereich und der zweiten Druckbereich derart komprimiert werden, dass sich die Summe der ersten Druckbereich und der zweiten Druckbereich während der Kompression zunimmt, um den ersten Gastrointestinaltraktabschnitt und den zweiten Gastrointestinaltraktabschnitt abzudichten,
**dadurch gekennzeichnet, dass** der erste Körper und der zweite Körper derart ausgebildet sind, dass sich die Summe der ersten Druckbereich und der zweiten Druckbereich während der Kompression derart vergrößert, dass die Muscularis-Schicht und/oder Mukosaschicht der Gastrointestinaltraktabschnitte zur Seite gedrückt werden und die Submukosaschichten der Gastrointestinaltraktabschnitte kontaktiert werden.

2. Chirurgisches Instrument nach Anspruch 1, wobei der erste Körper und/oder zweite Körper ein quellbares Hydrogel (22, 32) umfasst.

3. Chirurgisches Instrument nach Anspruch 2, das derart ausgebildet ist, dass das quellbare Hydrogel dazu ausgebildet ist, dort platziert zu werden, wo der erste Gastrointestinaltraktabschnitt und der zweite Gastrointestinaltraktabschnitt kontaktiert und abgedichtet sind, derart, dass das Hydrogel beim Quellen eine Kraft auf die abgedichteten Abschnitte ausübt und zu zusätzlichem Kontakt zwischen den Submukosaschichten führt, ohne die Vaskularisierung dieser zusätzlich kontaktierten Submukosaschichten zu beeinträchtigen, um eine Heilung durch primäre Absicht zu fördern.

4. Chirurgisches Instrument nach einem der Ansprüche 2 bis 3, wobei das quellbare Hydrogel in der Lage ist, in einem Menge von 0-100 Vol.-%, vorzugsweise 10-80 Vol.-%, stärker bevorzugt etwa 50 Vol.-%, bezogen auf das ursprüngliche Volumen des Hydrogels, zu quellen.

5. Chirurgisches Instrument nach einem der vorhergehenden Ansprüche, wobei der erste und/oder zweite Körper derart ausgebildet sind, dass sie den ersten Gastrointestinaltraktabschnitt und den zweiten Gastrointestinaltraktabschnitt zwischen einer geneigten ersten Druckbereich und/oder einer geneigten zweiten Druckbereich komprimieren.

6. Chirurgisches Instrument nach einem der vorhergehenden Ansprüche, wobei das chirurgische Instrument ein chirurgisches Klammernahtgerät ist, bei dem der erste Körper einen Amboss (40) umfasst und der zweite Körper ein Gehäuse (50) umfasst, das eine Klammerkartusche und eine Klammernahtgerätbasis umfasst, durch die Klammern gegen den Amboss getrieben werden können.

7. Chirurgisches Instrument nach einem der vorhergehenden Ansprüche, ferner umfassend ein Messer zum Erzeugen eines Schnitts in einem Schneidabschnitt der röhrenförmigen Gastrointestinaltraktabschnitte;
wobei der erste Körper und/oder der zweite Körper ferner derart ausgebildet sind, dass sich während der Verwendung die Summe der ersten Druckbereich und der zweiten Druckbereich in Richtung des Schneidabschnitts der röhrenförmigen Gastrointestinaltraktabschnitte vergrößert.

8. Chirurgisches Instrument nach einem der vorhergehenden Ansprüche, wobei das genannte chirurgische Klammernahtgerät ein chirurgisches zirkuläres Klammernahtgerät oder ein chirurgisches lineares Klammernahtgerät ist.

9. Chirurgisches Instrument nach einem der vorhergehenden Ansprüche, wobei das chirurgische Instrument zum Abstreifen der Submukosaschichten von den Muscularis- und/oder Mukosaschichten ausgebildet ist, während der erste Gastrointestinaltraktabschnitt und der zweite Gastrointestinaltraktabschnitt komprimiert sind.

10. Chirurgisches Instrument nach einem der vorhergehenden Ansprüche, wobei das chirurgische Instrument dazu ausgebildet ist, die Muscularis-Schichten (11) und Mukosaschichten (13) der Gastrointestinaltraktabschnitte zur Seite in Richtung des Lumens der Gastrointestinaltraktabschnitte zu drücken.

11. Chirurgisches Instrument nach einem der Ansprüche 1 bis 9, wobei das chirurgische Instrument dazu ausgebildet ist, die Muscularis-Schichten (11) und Mukosaschichten (13) der Gastrointestinaltraktabschnitte zur Seite in Richtung des Darms der Gastrointestinaltraktabschnitte zu drücken.

12. Chirurgisches Instrument nach einem der vorhergehenden Ansprüche, wobei während der Verwendung die erste Druckbereich und/oder zweite Druckbereich schräg oder geneigt in Bezug auf den ersten und den zweiten Gastrointestinaltraktabschnitt, die komprimiert werden sollen, positioniert werden kann.

13. Chirurgisches Instrument nach einem der vorhergehenden Ansprüche, wobei während der Verwendung, während der Kompression, die Muscularis-Schichten (11) und Mukosaschichten (13) zur Seite gedrückt werden und die Submukosaschichten kontaktiert werden, bis zu einem Punkt, an dem eine im Wesentlichen wasserdichte Abdichtung zwischen den Submukosaschichten erhalten wird.

14. Chirurgisches Instrument nach einem der vorhergehenden Ansprüche, wobei das chirurgische Instrument ein zirkuläres Klammernahtgerät ist und sich während der Verwendung die Summe der ersten Druckbereich und der zweiten Druckbereich in Richtung der Mitte des Querschnitts des Klammernahtgeräts vergrößert.

## Revendications

1. Instrument chirurgical pour réaliser une anastomose d'une première section de tractus gastro-intestinal et d'une deuxième section de tractus gastro-intestinal, ledit instrument comprenant :
un premier corps (20) ayant une première zone de pression (21) ;
un deuxième corps (30) ayant une deuxième zone de pression (31) ;
un moyen de raccordement (60) raccordant le premier corps et le deuxième corps,
dans lequel le moyen de raccordement est configuré pour déplacer le premier corps entre une position ouverte éloignée du deuxième corps et une position fermée à proximité du deuxième corps ;
dans lequel les premier et deuxième corps sont configurés de sorte que, pendant l'utilisation, la première section de tractus gastro-intestinal et la deuxième section de tractus gastro-intestinal sont comprimées entre la première zone de pression et la deuxième zone de pression de sorte que la somme de la première zone de pression et de la deuxième zone de pression augmente pendant la compression pour sceller la première section de tractus gastro-intestinal et la deuxième section de tractus gastro-intestinal,
**caractérisé en ce que** le premier corps et le deuxième corps sont configurés de sorte que la somme de la première zone de pression et de la deuxième zone de pression augmente pendant la compression de sorte que la couche musculaire et/ou la couche muqueuse des sections de tractus gastro-intestinal sont (est) écartée(s) et les couches sous-muqueuses des sections de tractus gastro-intestinal sont mises en contact.

2. Instrument chirurgical selon la revendication 1, dans lequel le premier corps et/ou le deuxième corps comprennent (comprend) un hydrogel capable de gonfler (22, 32).

3. Instrument chirurgical selon la revendication 2, configuré de sorte que l'hydrogel capable de gonfler est configuré pour être placé à l'endroit où la première section de tractus gastro-intestinal et la deuxième section de tractus gastro-intestinal sont en contact et scellées, de sorte que l'hydrogel, lors de son gonflement, exerce une force sur les sections scellées et se traduit par le contact supplémentaire entre les couches sous-muqueuses sans compromettre la vascularisation de ces couches sous-muqueuses mises davantage en contact, afin de favoriser la cicatrisation en première intention.

4. Instrument chirurgical selon l'une quelconque des revendications 2 à 3, dans lequel l'hydrogel capable de gonfler peut gonfler selon une quantité de 0 à 100% en volume, de préférence de 10 à 80% en volume, encore de préférence d'environ 50% en volume par rapport au volume d'origine de l'hydrogel.

5. Instrument chirurgical selon l'une quelconque des revendications précédentes, dans lequel le premier et/ou le deuxième corps sont (est) configuré(s) pour comprimer la première section de tractus gastro-intestinal et la deuxième section de tractus gastro-intestinal entre une première zone de pression inclinée et/ou une deuxième zone de pression inclinée.

6. Instrument chirurgical selon l'une quelconque des revendications précédentes, ledit instrument chirurgical étant une agrafeuse chirurgicale, dans lequel le premier corps comprend une enclume (40) et le deuxième corps comprend un boîtier (50) qui comprend une cartouche d'agrafes et une base d'agrafeuse par le biais de laquelle les agrafes peuvent être entraînées contre l'enclume.

7. Instrument chirurgical selon l'une quelconque des revendications précédentes, comprenant en outre un couteau pour créer une incision dans une section de coupe des sections de tractus gastro-intestinal tubulaires ;
dans lequel le premier corps et/ou le deuxième corps sont (est) en outre configuré(s) de sorte que pendant l'utilisation, la somme de la première zone de pression et de la deuxième zone de pression augmente dans la direction de la section de coupe des sections de tractus gastro-intestinal tubulaires.

8. Instrument chirurgical selon l'une quelconque des revendications précédentes, dans lequel ladite agrafeuse chirurgicale est une agrafeuse chirurgicale circulaire ou une agrafeuse chirurgicale linéaire.

9. Instrument chirurgical selon l'une quelconque des revendications précédentes, dans lequel l'instrument chirurgical est configuré pour enlever les couches sous-muqueuses des couches musculaires et/ou muqueuses alors que la première section de tractus gastro-intestinal et la deuxième section de tractus gastro-intestinal sont comprimées.

10. Instrument chirurgical selon l'une quelconque des revendications précédentes, dans lequel l'instrument chirurgical est configuré pour écarter les couches musculaires (11) et les couches muqueuses (13) des sections de tractus gastro-intestinal vers la lumière des sections de tractus gastro-intestinal.

11. Instrument chirurgical selon l'une quelconque des revendications 1 à 9, dans lequel l'instrument chirurgical est configuré pour écarter les couches musculaires (11) et les couches muqueuses (13) des sections de tractus gastro-intestinal vers la partie intestinale des sections de tractus gastro-intestinal.

12. Instrument chirurgical selon l'une quelconque des revendications précédentes, dans lequel, pendant l'utilisation, la première zone de pression et/ou la deuxième zone de pression peuvent (peut) être positionnée(s) de manière inclinée ou oblique par rapport aux première et deuxième sections de tractus gastro-intestinal qui doivent être comprimées.

13. Instrument chirurgical selon l'une quelconque des revendications précédentes, pendant l'utilisation, au cours de la compression, les couches musculaires (11) et les couches muqueuses (13) sont écartées et les couches sous-muqueuses sont en contact jusqu'à un point auquel une étanchéité sensiblement étanche à l'eau entre les couches sous-muqueuses est obtenue.

14. Instrument chirurgical selon l'une quelconque des revendications précédentes, dans lequel l'instrument chirurgical est une agrafeuse circulaire et pendant l'utilisation, la somme de la première zone de pression et de la deuxième zone de pression augmente dans la direction du centre de la section transversale de l'agrafeuse.
